# EUROPEAN PATENT APPLICATION

(11) **EP 1 598 070 A1**
(43) Date of publication of application: **23.11.2005**
(21) Application number: 04291119.8
(22) Date of filing: 30.04.2004
(51) Int. Cl.: A61K 31/727, A61P 9/10

(54) **Administration of enoxaparin sodium to patients 75 years and older with ST-segment elevation myocardial infarction**

(71) Applicant: Aventis Pharma S.A., 92160 Antony (FR)
(72) Inventor: Sanderink, Ger-Jan C.M. Aventis Pharma S.A., 92160 Antony (FR); Vetticaden, Santosh Aventis Pharma S.A., 92160 Antony (FR); Bacher, Hans Peter Aventis Pharma S.A., 92160 Antony (FR)
(74) Representative: Rousseau, Pierrick Edouard

(57) **Abstract**

Methods for treating ST-segment elevation myocardial infarction in a human patient 75 years of age or older. The methods comprise administering a dose of less than 1 mg per kg body weight, about .75 mg per kg of body weight, or .75 mg per kg of body weight of enoxaparin sodium by subcutaneous injection approximately every twelve hours for a therapeutic dosing period. The methods may include fibrinolytic therapy. The treatment methods may be used to prevent one or more of, mortality, myocardial re-infarction, myocardial ischemia, stroke, or severe congestive heart failure. Articles of manufacture for use in connection with treating ST-segment elevation myocardial infarction in a human patient 75 years of age or older are also disclosed. The articles of manufacture comprise enoxaparin sodium, and instructions designed to achieve administration to a patient of a dose of less than 1 mg per kg body weight, about .75 mg per kg of body weight, or .75 mg per kg of body weight of enoxaparin sodium, by subcutaneous injection approximately every twelve hours for a therapeutic dosing period.

## Description

Broadly, this invention is directed to novel methods for treating ST-segment elevation myocardial infarction in a human patient 75 years of age or older. The methods comprise administering enoxaparin sodium (sometimes referred to herein as "enoxaparin") to the human patient. The methods may also comprise administering fibrinolytic therapy, which may comprise administering one or more fibrinolytic selected from streptokinase, alteplase, tenecteplase, and reteplase to the human patient. The invention also relates to methods for treating ST-segment elevation myocardial infarction in a human patient 75 years of age or older, where the treatment is characterized by prevention of one or more of mortality, myocardial re-infarction, myocardial ischemia, stroke, or severe congestive heart failure in the human patient. This invention also provides articles of manufacture for use in connection with a patient 75 years of age or older with ST-segment elevation myocardial infarction, comprising enoxaparin and instructions designed to achieve administration of the enoxaparin.

Enoxaparin sodium is available from Aventis under the trademark Lovenox® (Clexane® in some other countries). Enoxaparin sodium exhibits renal clearance. Study data have demonstrated a significant impact of renal function on enoxaparin pharmacokinetics as measured by plasma anti-Xa activity. The main effect of renal impairment is a reduced clearance, which results in a significantly longer elimination half-life and increased exposure to the drug. This in turn results in higher pre-dose levels after repeated administration and then an increase in Amax (maximum observed activity) after repeated dosing.

Patients 75 years of age or older often exhibit renal impairment. Elevated levels of enoxaparin sodium in patients with renal impairment may cause undesirable effects, such as bleeding resulting from excessive anticoagulation. In order to overcome this unwanted side effect in use of enoxaparin sodium, new dosing regimens are desired, which reduce or eliminate the increased risk of bleeding in patients 75 years of age or older.

While the possibility of a dose adjustment in these patients has been suggested, there is a difficulty, however, in determining the appropriate amount of any adjustment in such patients. This difficulty is the result of the need to balance the desired antithrombotic activity of enoxaparin sodium against the possibility of excessive bleeding or other undesired results of accumulation of enoxaparin sodium. Furthermore, selection of the appropriate dose must be accompanied by consideration for age-related changes in clearance and distribution of enoxaparin, and with respect to changes in the tolerance to antithrombotic therapy.

Treatment of ST-segment elevation myocardial infarction in patients 75 years of age or older, by administering a dose of .75 mg per kg of body weight of enoxaparin sodium by subcutaneous injection approximately every twelve hours possibly may reduce the incidence of major bleeding in these patients.

As used herein "enoxaparin sodium" refers to the low molecular weight heparin (LMWH) approved by the U.S. Food and Drug Administration (FDA), or any other regulatory agency outside of the United States, as Lovenox® (enoxaparin sodium injection), Clexane® or Klexane®, and any LMWH approved by the FDA, or any other regulatory agency outside of the United States, pursuant to an application citing Lovenox® (enoxaparin sodium injection), Clexane® or Klexane® as the listed drug. Enoxaparin sodium is available from Aventis and sold in the United States in the form of enoxaparin sodium injection, under the trademark Lovenox® (Clexane® in some other countries). In general, enoxaparin sodium is obtained by alkaline degradation of heparin benzyl ester derived from porcine intestinal mucosa. Its structure is characterized, for example, by a 2-0-sulfo-4-enepyranosuronic acid group at the nonreducing end and a 2-N,6-0-disulfo-D-glucosamine at the reducing end of the chain. The average molecular weight is about 4500 daltons. The molecular weight distribution is:

| | |
|---|---|
| <2000 daltons | ≤ 20% |
| 2000 to 8000 daltons | ≥ 68% |
| >8000 daltons | ≤ 18% |

Enoxaparin sodium injection is a sterile aqueous solution containing enoxaparin sodium. Enoxaparin sodium injection is available from Aventis at 100 mg/ml in prefilled syringes (30 mg/0.3 mL pre-filled syringes, 40 mg/0.4 mL pre-filled syringes, 60 mg/0.6 mL pre-filled syringes, 80 mg/0.8 mL pre-filled syringes, and 100 mg/1.0 mL pre-filled syringes), graduated prefilled syringes, multiple-dose vials (300 mg/3.0 mL multi-dose vials), and ampoules (30 mg/0.3 mL). Enoxaparin sodium injection 100 mg/mL concentration contains 10 mg enoxaparin sodium (approximate anti-Factor Xa activity of 1000 IU [with reference to the W.H.O. First International Low Molecular Weight Heparin Reference Standard]) per 0.1 mL water for injection. Enoxaparin sodium injection is also available from Aventis at 150 mg/ml in graduated prefilled syringes (90 mg/0.6 mL pre-filled syringes, 120 mg/0.8 mL pre-filled syringes, and 150 mg/1.0 mL pre-filled syringes). Enoxaparin sodium injection 150 mg/mL concentration contains 15 mg enoxaparin sodium (approximate anti-Factor Xa activity of 1500 IU [with reference to the W.H.O. First International Low Molecular Weight Heparin Reference Standard]) per 0.1 mL water for injection.

The enoxaparin sodium injection prefilled syringes and graduated prefilled syringes are preservative-free and intended for use only as a single-dose injection. The multiple-dose vial contains 15 mg/1.0 mL benzyl alcohol as a preservative. The pH of the injection is 5.5 to 7.5. Enoxaparin sodium injection may also be administered in an arterial line for a hemodialysis indication.

As used herein, reference to administration of enoxaparin sodium by subcutaneous injection approximately every twelve hours means, for example, administration every twelve hours plus or minus two hours. Similarly, reference to administration of enoxaparin sodium by subcutaneous injection approximately every twenty-four hours means, for example, administration every twenty-four hours plus or minus two hours.

As used herein, the term "treat," "treating" or "treatment" refers to the administration of therapy to an individual who already manifests at least one symptom of a disease or condition (e.g., ST-segment elevation myocardial infarction), or who has previously manifested at least one symptom of a disease or condition.

The term "prevent," "preventing" and "prevention" refers to the administration of therapy an individual who may ultimately manifest at least one symptom of a disease or condition (e.g., myocardial ischemia) but who has not yet done so, to reduce the chance that the individual will develop the symptom of a disease or condition over a given period of time. Such a reduction may be reflected, for example, in a delayed onset of the at least one symptom of a disease or condition in the patient.

A single therapy may simultaneously treat and prevent one or more of the same or different conditions. As a non-limiting example, individuals having ST-segment elevation myocardial infarction have a higher risk of mortality, of developing, myocardial re-infarction, myocardial ischemia, stroke, or severe congestive heart failure. Therapy administered to treat ST-segment elevation myocardial infarction in these patients may also act to prevent mortality, and/or development of one or more of myocardial re-infarction, myocardial ischemia, stroke, or severe congestive heart failure.

As used herein, "glycoprotein IIb/IIIa inhibitor" or "GP IIb/IIIa inhibitor" means a compound that antagonizes the fibrinogen receptor on platelets. Platelet aggregation involves activation of the glycoprotein IIb/IIIa receptor on the surface of platelets. The IIb/IIIa receptors of adjacent platelets bind fibrinogen molecules to cross link platelets, and the cross linking initiates thrombus formation. Antagonism of the IIb/IIIa receptor inhibits platelet aggregation and retards thrombus formation at sites of plaque rupture. Commercially available GP IIb/IIIa inhibitors include abciximab, eptifibatide, and tirofiban.

As used herein, "therapeutic dosing period" refers to the period of time beginning with the administration of a first dose of enoxaparin sodium and ending with the administration of the last dose of enoxaparin sodium on a continuing basis. For example, if enoxaparin sodium is administered approximately every twelve hours for a total of nine doses, the therapeutic dosing period is approximately 96 hours, the time between the first dose and the ninth.

As used herein, "ST-segment elevation myocardial infarction" refers to myocardial infarction characterized by an ST-segment elevation of 0.1 mV in two or more limb leads, or 0.2 mV in two or more contiguous precordial leads, or left bundle-branch block.

As used herein, "mortality" refers to death resulting from any cause (i.e., all-cause mortality). Death may be classified into two categories, cardiovascular and non-cardiovascular. Cardiovascular death may be further classified as cardiac death or other cardiovascular death. Cardiac death has a cardiac cause as the main reason of death. All other cardiovascular deaths are other cardiovascular death (e.g., a stroke, bleeding episode, pulmonary embolism, or procedural caused). Non-cardiovascular death is any death due to a clearly documented non-cardiovascular cause (e.g., malignancy, trauma, or infection).

As used herein, "percutaneous coronary intervention" (or "PCI") refers to any technique capable of relieving coronary narrowing, including but not limited to balloon angioplasty, rotational atherectomy, directional atherectomy, extraction atherectomy, laser angioplasty, and implantation of intracoronary stents and other catheter devices for treating coronary atherosclerosis.

If a patient already being treated for ST-segment elevation myocardial infarction by administration of enoxaparin sodium by subcutaneous injection is treated by PCI, administration of enoxaparin sodium by subcutaneous injection to said patient will be discontinued. If more than eight hours have passed since the patient's previous subcutaneous dose of enoxaparin sodium and the initiation of PCI, an enoxaparin sodium dosage of 0.3 mg/kg body weight may be administered to said patient by I.V. bolus before initiation of PCI.

As used herein, "myocardial re-infarction" refers to any myocardial infarction (MI) that occurs after and is distinct from an ST-segment elevation myocardial infarction being treated. By way of example, and without limitation, myocardial re-infarction, as used herein, includes recurrent acute myocardial infarction, which may be defined by, for example, any one or more of criteria A-E below. In the descriptions of criteria A-E, an asterisk (*) indicates that the following special circumstances must be considered when evaluating a possible recurrent MI: (1) ST-depression in V1-V3 will be considered equivalent to ST-elevation if the recurrent MI is suspected to be true posterior in location; and (2) An increase in Rw amplitude in V1-V3 will be considered equivalent to Q-waves if the recurrent MI is suspected to be true posterior in location.
**A. Within 18 hours of symptoms onset of the index MI, a patient is considered to have myocardial re-infarction if the patient exhibits:**
   (1) New or re-elevation* of ST-segments ≥ 0.1 mV (80 msec after the J-point) in ≥ 2 contiguous precordial leads or ≥ 2 adjacent limb leads; and
   (2) At least one of the following:
      (a) Recurrent ischemic discomfort ≥ 20 minutes at rest; or
      (b) ischemia-mediated new hemodynamic decompensation requiring treatment with at least one of the following: IV inotropes or IABP.
**B. After 18 hours of symptoms onset of index MI but before the cardiac biomarker assayed has returned to normal, a patient is considered to have myocardial re-infarction if the patient exhibits:**
   (1) Cardiac biomarker re-elevation defined as:
      (a) An increase by at least 50% over the previous value; and
      (b) Documentation that the cardiac biomarker was decreasing prior to the suspected recurrent MI; and
   (2) At least one of the following additional supportive criteria:
      (a) New ischemic discomfort >/= 20 minutes at rest;
      (b) new hemodynamic decompensation requiring treatment with at least one of the following: IV inotropes or IABP; or
      (c) One of the following ECG changes:
         (i) New or re-elevation of ST segments* > 0.2 mV (80 msec after the J-point) in > 2 contiguous precordial leads or > 0.1 mV in > 2 adjacent limbleads;
         (ii) Development of new, abnormal Q-waves* (> 30 msec in duration and > 0.1 mV in depth) in > 2 contiguous precordial leads or > 2 adjacent limb leads considered to be distinct from the evolution of the index MI; or (iii) New left bundle branch block; or
   (3) Pathologic findings of a new acute MI felt to be distinct from the index MI.
**C. After 18 hours of symptoms onset of index MI and after cardiac biomarker assayed has been documented to return to normal, a subject must meet criteria (1) and (2) or criterion (3) alone or criterion (4) alone:**
   (1) Typical cardiac biomarker rise and fall with the following degrees of elevation accepted as biochemical evidence of myocardial necrosis:
      (a) CK-MB: maximal concentration > upper limit of normal (ULN); or
      (b) Total CK: maximal concentration > 2x ULN. (This applies only if the CK-MB or troponin are not available since they supercede total CK); and
      (c) Troponin T or I: maximal concentration greater than the cutpoint for MI; and
   (2) At least one of the following additional supportive criteria:
      (a) Ischemic discomfort or equivalent at rest lasting ≥ 20 minutes; or
      (b) ST-Tw changes indicative of ischemia*; or
   (3) Development of new, abnormal Q waves* (> 30 msec in duration and > 0.1 mV in depth) in > 2 contiguous precordial leads or > 2 adjacent limb leads, considered to be distinct from the evolution of the index MI; or
   (4) Pathologic findings of a new acute MI felt to be distinct from the index MI.
**D. Within 24 hours after PCI, a patient must have EITHER:**
   (1) CK-MB (or total CK if CK-MB not available) > 3x ULN and, if the pre-PCI CK-MB (or total CK) was > ULN, both an increase by at least 50% over the previous value and documentation that CK-MB (or total CK) was decreasing prior to the suspected recurrent MI; or
   (2) Development of new abnormal Q waves * (> 30 msec in duration and > 1 mV in depth) in > 2 contiguous precordial leads or > 2 adjacent limb leads, considered to be distinct from the evolution of the index MI; or
   (3) Pathologic findings of a new acute MI felt to be distinct from the index MI.
   (Symptoms are not required.)
**E. Within 24 hours after CABG a patient must have at least one of the following 3 criteria:**
   (1) CK-MB > 10x ULN and , if the pre-CABG CK-MB was > ULN, both an increase by at least 50% over the previous value and documentation that CK-MB was decreasing prior to the suspected recurrent MI; or
   (2) CK-MB > 5x ULN and if the pre-CABG CK-MB was > ULN, both an increase by atleast 50% over the previous value and documentation that CK-MB was decreasing prior to the suspected recurrent MI; and
      Development of new, abnormal Q-waves* (> 30 msec in duration and > 1 MV in depth) in > 2 contiguous precordial leads or > 2 adjacent limb leads, considered to be distinct from the evolution of the index MI; or
   (3) Pathologic findings of a new acute MI felt to be distinct from the index MI.
   (Symptoms are not required. Evidence of new RWMA(s) may be considered if available.)

[020] As used herein, "myocardial ischemia" refers to a condition characterized by local anemia due to mechanical obstruction of the blood supply to the myocardium.

[021] Myocardial ischemia includes, for example, "recurrent myocardial ischemia requiring urgent revascularization." Recurrent myocardial ischemia requiring urgent revascularization is defined as an episode of recurrent myocardial ischemia at rest and/or necessitating hospitalization that leads to a revascularization procedure performed on the same hospitalization. This may include both the index hospitalization and any re-hospitalization following discharge for the index event. Recurrent ischemia requiring urgent revascularization is distinct from rescue PCI, which is PCI occurring within about 8 hours of administration of a first dose of enoxaparin sodium, and not preceded by a recurrent ischemic event or a recurrent myocardial infarction.

[022] As used herein, "stroke" refers to the new onset of focal or global neurological deficit caused by ischemia or hemorrhage within or around the brain and lasting for more than 24 hours. Intracranial hemorrhage may be diagnosed by appropriate brain imaging, including, for example, a CT or MRI that shows the presence of an acute blood mass. Ischemic or bland infarction may be diagnosed, for example, by appropriate imaging which shows the presence of one or more of the following: hypodensity, edema, midline shift or ventricular effacement without evidence of hemorrhage. Hemorrhagic conversion of an ischemic infarction may be diagnosed by appropriate imaging that shows an ischemic infarction with localized petechial or confluent bleeding into necrotic tissue. The functional status scale (modified Rankin scale) may be used to classify patients with stroke as follows:
0 = no symptoms
1 = symptoms but no signs of disability.
2 = slight disability (activities of daily life without assistance).
3 = moderate disability (requires some assistance; able to walk).
4 = moderately severe disability (requires assistance for walking & activities of daily life).
5 = severe disability (bedridden).
Using this scale, a disabling stroke may be defined as characterized by a categorization into category 4 or 5.

[023] As used herein, "severe congestive heart failure" refers to congestive heart failure characterized by rales over more than 50% of the lung fields that do not clear with coughing or evidence of pulmonary edema on chest radiograph.

[024] As used herein, "fibrinolytic therapy" refers to therapy designed to lead to the dissolution of fibrin in blood clots by, for example, hydrolosis. Fibrinolytic therapy includes, without limitation, administration of a fibrinolytic such as streptokinase, alteplase, tenecteplase, or reteplase.

[025] As used herein, "streptokinase" refers to a bacterial protein elaborated by group C (beta)-hemolytic streptococci. Streptokinase is available from Aventis and sold in the United States under the trademark Streptase®. Dosing and other additional information regarding streptokinase is publicly available, for example, on the FDA approved Streptase® label, and known to one of skill in the art.

[026] As used herein, "alteplase" refers to a form of human tissue plasminogen activator (tPA). Alteplase is a tissue plasminogen activator (t-PA) produced by recombinant DNA technology. It is a sterile, purified glycoprotein of 527 amino acids. It is synthesized using the complementary DNA (cDNA) for natural human tissue-type plasminogen activator (t-PA) obtained from an established human cell line. Alteplase is available from Genentech and sold in the United States under the trademarks Activase® and Cathflo™ Activase®. Dosing and other additional information regarding alteplase is publicly available, for example, on the FDA approved Activase® and Cathfio™ Activase® labels, and known to one of skill in the art.

[027] As used herein, "tenecteplase" refers to a tissue plasminogen activator (tPA) produced by recombinant DNA technology. Tenecteplase is a 527 amino acid glycoprotein developed by introducing the following modifications to the complementary DNA (cDNA) for natural human tPA: a substitution of threonine 103 with asparagine, and a substitution of asparagine 117 with glutamine, both within the kringle 1 domain, and a tetra-alanine substitution at amino acids 296-299 in the protease domain. Tenecteplase produced from recombinant sources is available from Genentech and sold in the United States under the trademark TNKase™. Dosing and other additional information regarding tenecteplase is publicly available, for example, on the FDA approved TNKase™ label, and known to one of skill in the art.

[028] As used herein, "reteplase" refers to is a non-glycosylated deletion mutein of tissue plasminogen activator (tPA), containing the kringle 2 and the protease domains of human tPA. Reteplase contains 355 of the 527 amino acids of native tPA (amino acids 1-3 and 176-527). Reteplase is produced by recombinant DNA technology in *E. coli.* The protein is isolated as inactive inclusion bodies from *E. coli*, converted into its active form by an *in vitro* folding process and purified by chromatographic separation. The molecular weight of Reteplase is 39,571 daltons. Reteplase is available from Centecor in the United States under the trademark Retavase®. Dosing and other additional information regarding reteplase is publicly available, for example, on the FDA approved Retavase® label, and known to one of skill in the art.

[029] "Body weight" refers to the weight of a patient that is determined by actual weighing or by estimation prior to administration of enoxaparin sodium. In the event that the body weight of a patient is estimated prior to administration of the first dose of enoxaparin sodium, the body weight of the patient may be subsequently determined by actual weighing before any subsequent dose of enoxaparin, and the amount of enoxaparin administered to the patient with the next subsequent dose adjusted accordingly.

[030] Because creatinine is found in stable plasma concentrations, is freely filtered and not reabsorbed, and is minimally secreted by the kidneys, creatinine clearance is used as the standard by which kidney function is assessed. The creatinine clearance test compares the level of creatinine in urine with the creatinine level in the blood. Clearance is often measured as milliliters/minute (mUmin). Creatinine clearance values may be calculated by the Cockroft-Gault formula. (Cockcroft and Gault, Nephron, Vol. 16, pp. 31-41 (1976)). In a normal human subject, creatinine clearance is > 80 mL/min.

[031] As used herein, "renal impairment" is a condition characterized by a creatinine clearance rate of ≤ 80 mL/min. Patients with renal impairment may be referred to herein as having renal insufficiency or as renally impaired patients.

[032] As used herein, "severe renal impairment" means an impairment of renal function in a patient characterized by a creatinine clearance rate of < 30 mL/min. Patients with severe renal impairment may be referred to herein as having severe renal insufficiency or as severely (or severe) renally impaired patients.

[033] In an embodiment, the invention provides a method for treating ST-segment elevation myocardial infarction in a human patient 75 years of age or older, comprising administering to said patient a dose of less than 1 mg per kg of body weight of enoxaparin sodium by subcutaneous injection approximately every twelve hours for a therapeutic dosing period. In another embodiment of the method, about .75 mg per kg of body weight of enoxaparin sodium is administered approximately every twelve hours. In another embodiment of the method, .75 mg per kg of body weight of enoxaparin sodium is administered approximately every twelve hours. In a further embodiment, no GP IIb/IIIa inhibitor is administered to the patient during the therapeutic dosing period. In another embodiment of the method, the therapeutic dosing period is for approximately 8 days or until discharge from the hospital, whichever is less. In another embodiment of the method, the therapeutic dosing period comprises administration of at least three doses of enoxaparin sodium. In another embodiment of the method, the therapeutic dosing period is until approximately twelve hours or less before the patient undergoes PCI. In such a patient, a dosage of about 0.3 mg/kg enoxaparin sodium may be administered by I.V. bolus before initiation of PCI if more than eight hours have passed since the patient's previous subcutaneous dose of enoxaparin sodium. In a further embodiment of the method, the first two doses of enoxaparin sodium are equal to or less than a maximum dose of enoxaparin sodium; for example, when a dose of .75 mg per kg of body weight of enoxaparin sodium is administered approximately every twelve hours, each of the first two doses may be .75 mg per kg of body weight, or 75 mg, whichever is less. In a further embodiment an initial dose of enoxaparin sodium is administered by I.V. bolus prior to or concurrently with the initial subcutaneous dosage of enoxaparin sodium. This initial I.V. bolus dosage may, for example, be a dose of about 30 mg of enoxaparin sodium. Alternatively, no initial I.V. bolus of enoxaparin sodium is administered. In a further embodiment of the method, the method further comprises administration of aspirin to said patient. For example, about 150 mg to about 325 mg of non-enteric coated aspirin may be administered orally or about 500 mg of aspirin may be administered intravenously after said patient is identified with ST-segment elevation myocardial infarction, and then doses of between about 75 to about 325 mg (coated or uncoated) aspirin may be administered once daily thereafter for a minimum of 30 days. In a further embodiment of the invention, the method further comprises administering fibrinolytic therapy to said patient. Fibrinolytic therapy may comprise, for example, administering one or more fibrinolytic selected from streptokinase, alteplase, tenecteplase, and reteplase. In an embodiment, the first dose of enoxaparin sodium is administered approximately 15 minutes before to approximately 30 minutes after the initiation of fibrinolytic therapy. In a further embodiment of the invention, the method further comprises prevention of one or more of, for example, mortality, myocardial re-infarction, myocardial ischemia, stroke, or severe congestive heart failure.

[034] In a further embodiment, the invention provides a method for treating ST-segment elevation myocardial infarction in a human patient 75 years of age or older, comprising administering to said patient at least one dose of about .75 mg per kg of body weight of enoxaparin sodium by subcutaneous injection, determining the renal function of the patient and, if the patient is determined to have severe renal impairment, adjusting the dose of enoxaparin administered to said patient to 1 mg per kg of body weight of enoxaparin sodium administered to the patient approximately every 24 hours throughout the remainder of the therapeutic dosing period. In another embodiment, fibrinolytic therapy is administered to said patient. In a further embodiment, the fibrinolytic therapy comprises administering one or more fibrinolytic selected from streptokinase, alteplase, tenecteplase, and reteplase. In another embodiment, a first dose of enoxaparin sodium is administered from approximately 15 minutes before to approximately 30 minutes after the initiation of fibrinolytic therapy. A further embodiment comprises administering a dosage of about 0.3 mg/kg enoxaparin sodium to said patient by I.V. bolus before initiation of PCI if more than eight hours have passed since the patient's previous subcutaneous dose of enoxaparin sodium.

[035] In another embodiment, the invention provides a method for treating ST-segment elevation myocardial infarction in a human patient 75 years of age or older, comprising administering to said patient a dose of .75 mg per kg of body weight of enoxaparin sodium by subcutaneous injection approximately every twelve hours for approximately 8 days or until discharge from the hospital, whichever is less; wherein each of the first two doses of enoxaparin sodium are .75 mg per kg of body weight, or 75 mg, whichever is less; wherein said treatment further comprises administering fibrinolytic therapy comprising administering one or more fibrinolytic selected from streptokinase, alteplase, tenecteplase, and reteplase; wherein said treatment further comprises administering about 150 mg to about 325 mg of non-enteric coated aspirin orally or about 500 mg intravenously after said patient is identified with ST-segment elevation myocardial infarction, and administration of aspirin doses of between about 75 to about 325 mg (coated or uncoated) aspirin once daily thereafter for a minimum of 30 days. In a further embodiment said treatment comprises prevention of one or more of mortality, myocardial re-infarction, myocardial ischemia, stroke, or severe congestive heart failure.

[036] In another embodiment, the invention provides a method for treating ST-segment elevation myocardial infarction in a human patient 75 years of age or older, comprising administering to said patient a dose of .75 mg per kg of body weight of enoxaparin sodium by subcutaneous injection approximately every twelve hours for a therapeutic dosing period; wherein each of the first two doses of enoxaparin sodium are .75 mg per kg of body weight, or 75 mg, whichever is less; determining the renal function of the patient and, if the patient is determined to have severe renal impairment, adjusting the dose of enoxaparin administered to said patient to 1 mg per kg of body weight of enoxaparin sodium administered to the patient approximately every 24 hours throughout the remainder of a therapeutic dosing period; wherein said treatment further comprises administering fibrinolytic therapy comprising administering one or more fibrinolytic selected from streptokinase, alteplase, tenecteplase, and reteplase; wherein said treatment further comprises administering about 150 mg to about 325 mg of non-enteric coated aspirin orally or about 500 mg intravenously as soon as said patient is identified with ST-segment elevation myocardial infarction, and administration of doses of between about 75 to about 325 mg (coated or uncoated) aspirin once daily thereafter for a minimum of 30 days. In a further embodiment said treatment comprises prevention of one or more of mortality, myocardial re-infarction, myocardial ischemia, stroke, or severe congestive heart failure.

[037] In another embodiment, the invention provides a method for treating ST-segment elevation myocardial infarction in a human patient 75 years of age or older, comprising administering to said patient a dose of .75 mg per kg of body weight of enoxaparin sodium by subcutaneous injection approximately every twelve hours until the patient undergoes PCI; wherein each of the first two doses of enoxaparin sodium are .75 mg per kg of body weight, or 75 mg, whichever is less; wherein a dosage of 0.3 mg/kg enoxaparin sodium is administered to said patient by I.V. bolus before initiation of PCI if more than eight hours have passed since the patient's previous subcutaneous dose of enoxaparin sodium; wherein said treatment further comprises administering fibrinolytic therapy comprising administering one or more fibrinolytic selected from streptokinase, alteplase, tenecteplase, and reteplase; wherein said treatment further comprises administering about 150 mg to about 325 mg of non-enteric coated aspirin orally or about 500 mg intravenously as soon as said patient is identified with ST-segment elevation myocardial infarction, and administration of aspirin doses of between about 75 to about 325 mg (coated or uncoated) once daily thereafter for a minimum of 30 days. In a further embodiment said treatment comprises prevention of one or more of mortality, myocardial re-infarction, myocardial ischemia, stroke, or severe congestive heart failure.

[038] Articles of manufacture for use in treating ST-segment elevation myocardial infarction in a human patient 75 years of age or older are also provided. The articles of manufacture comprise enoxaparin sodium, and instructions for administering enoxaparin sodium to a patient in connection with treating ST-segment elevation myocardial infarction in said patient in accordance with a method of the invention.

[039] In another embodiment the invention provides an article of manufacture for use in connection with treating ST-segment elevation myocardial infarction in a human patient 75 years of age or older, comprising enoxaparin sodium and instructions for the use of said enoxaparin sodium, said instructions being designed to achieve administration to said patient of less than 1 mg per kg of body weight of enoxaparin sodium by subcutaneous injection approximately every twelve hours for a therapeutic dosing period. In another embodiment, the instructions are designed to achieve administration to said patient of about .75 mg per kg of body weight of enoxaparin sodium is administered approximately every twelve hours. In another embodiment, the instructions are designed to achieve administration to said patient of .75 mg per kg of body weight of enoxaparin sodium is administered approximately every twelve hours. In another embodiment, the instructions are further designed to achieve administration to said patient of fibrinolytic therapy. Fibrinolytic therapy may comprise, for example, administration of one or more fibrinolytic selected from streptokinase, alteplase, tenecteplase, and reteplase. In another embodiment, the first dose of enoxaparin sodium is administered approximately 15 minutes before to approximately 30 minutes after the initiation of fibrinolytic therapy. In another embodiment, each of the first two doses of enoxaparin sodium are .75 mg per kg of body weight, or 75 mg, whichever is less. In another embodiment, the instructions are further designed to achieve administration of an initial dose of enoxaparin sodium by I.V. bolus prior to or concurrently with the initial subcutaneous dosage of enoxaparin sodium. This initial I.V. bolus dosage may, for example, be a dose of about 30 mg of enoxaparin sodium. Alternatively, the instructions may be designed so that no initial I.V. bolus of enoxaparin sodium is administered. In a further embodiment, the invention provides an article of manufacture comprising instructions designed to achieve administration to said patient of about 150 mg to about 325 mg of non-enteric coated aspirin orally or about 500 mg intravenously after said patient is identified with ST-segment elevation myocardial infarction, and administration of doses of between about 75 to about 325 mg (coated or uncoated) once daily thereafter for a minimum of 30 days. In another embodiment, the therapeutic dosing period is approximately 8 days or until discharge from the hospital, whichever is less. In another embodiment, the therapeutic dosing period is for a minimum of three doses. In another embodiment, the therapeutic dosing period is until approximately twelve hours our less prior to PCI therapy. In a further embodiment, said instructions are designed to achieve administration to said patient of a dosage of 0.3 mg/kg enoxaparin sodium to said patient by I.V. bolus before initiation of PCI if more than eight hours have passed since the patient's previous subcutaneous dose of enoxaparin sodium and the initiation of PCI. In a further embodiment, said treatment comprises prevention of one or more of mortality, myocardial re-infarction, myocardial ischemia, stroke, or severe congestive heart failure. In a further embodiment of the article of manufacture, said instructions are designed to achieve administration of enoxaparin sodium to said patient without a GP IIb/IIIa inhibitor being administered to said patient during the therapeutic dosing period. In a further embodiment, the invention provides an article of manufacture, wherein said instructions are further designed to achieve determining the renal function of the patient and, if the patient is determined to have severe renal impairment, adjusting the dose of enoxaparin administered to said patient to 1 mg per kg of body weight of enoxaparin sodium administered to the patient approximately every 24 hours throughout the remainder of the therapeutic dosing period.

[040] In another embodiment, the invention provides an article of manufacture for use in treating ST-segment elevation myocardial infarction in a human patient 75 years of age or older, comprising enoxaparin sodium and instructions for the use of said enoxaparin sodium, said instructions being designed to achieve administration to said patient of at least one dose of .75 mg per kg of body weight of enoxaparin sodium by subcutaneous injection, determining the renal function of the patient and, if the patient is determined to have severe renal impairment, adjusting the dose of enoxaparin administered to said patient to 1 mg per kg of body weight of enoxaparin sodium administered to the patient approximately every 24 hours throughout the remainder of the therapeutic dosing period. In another embodiment, the instructions are further designed to achieve administration of fibrinolytic therapy to said patient. In another embodiment, the fibrinolytic therapy comprises administering one or more fibrinolytic selected from streptokinase, alteplase, tenecteplase, and reteplase. In another embodiment, a first dose of enoxaparin sodium is administered approximately 15 minutes before to approximately 30 minutes after the initiation of fibrinolytic therapy. In a further embodiment, the instructions are further designed to achieve administration of a dosage of 0.3 mg/kg enoxaparin sodium to said patient by I.V. bolus before initiation of PCI if more than eight hours have passed since the patient's previous subcutaneous dose of enoxaparin sodium.

[041] In another embodiment, the invention provides an article of manufacture for use in connection with treating ST-segment elevation myocardial infarction in a human patient 75 years of age or older, comprising enoxaparin sodium and instructions for the use of said enoxaparin sodium, said instructions being designed to achieve administration to said patient of .75 mg per kg of body weight of enoxaparin sodium by subcutaneous injection approximately every twelve hours, for approximately 8 days or until discharge from the hospital, whichever is less; wherein each of the first two doses of enoxaparin sodium are .75 mg per kg of body weight, or 75 mg, whichever is less; wherein said treatment further comprises administering fibrinolytic therapy comprising administering one or more fibrinolytic selected from streptokinase, alteplase, tenecteplase, and reteplase; wherein said treatment further comprises administering about 150 mg to about 325 mg of non-enteric coated aspirin orally or about 500 mg intravenously after said patient is identified with ST-segment elevation myocardial infarction, and administration of doses of between about 75 to about 325 mg (coated or uncoated) once daily thereafter for a minimum of 30 days. In a further embodiment said treatment comprises prevention of one or more of mortality, myocardial re-infarction, myocardial ischemia, stroke, or severe congestive heart failure.

[042] In another embodiment, the invention provides an article of manufacture for use in connection with treating ST-segment elevation myocardial infarction in a human patient 75 years of age or older, comprising enoxaparin sodium contained within a syringe and instructions for the use of said enoxaparin sodium, said instructions being designed to achieve administration to said patient of .75 mg per kg of body weight of enoxaparin sodium subcutaneous injection approximately every twelve hours for a therapeutic dosing period; wherein each of the first two doses of enoxaparin sodium are .75 mg per kg of body weight, or 75 mg, whichever is less; wherein said instructions are further designed to achieve determining the renal function of the patient and, if the patient is determined to have severe renal impairment, adjusting the dose of enoxaparin administered to said patient to 1 mg per kg of body weight of enoxaparin sodium administered to the patient approximately every 24 hours throughout the remainder of the therapeutic dosing period; wherein said treatment further comprises administering fibrinolytic therapy comprising administering one or more fibrinolytic selected from streptokinase, alteplase, tenecteplase, and reteplase; wherein said treatment further comprises administering about 150 mg to about 325 mg of non-enteric coated aspirin orally or about 500 mg intravenously after said patient is identified with ST-segment elevation myocardial infarction, and administration of doses of between about 75 to about 325 mg (coated or uncoated) once daily thereafter for a minimum of 30 days; and wherein said treatment comprises prevention of one or more of mortality, myocardial re-infarction, myocardial ischemia, stroke, or severe congestive heart failure.

[043] In another embodiment, the invention provides an article of manufacture for use in connection with treating ST-segment elevation myocardial infarction in a human patient 75 years of age or older, comprising enoxaparin sodium and instructions for the use of said enoxaparin sodium, said instructions being designed to achieve administration to said patient of .75 mg per kg of body weight of enoxaparin sodium by subcutaneous injection approximately every twelve hours until the patient undergoes PCI; wherein each of the first two doses of enoxaparin sodium are .75 mg per kg of body weight, or 75 mg, whichever is less; wherein a dosage of 0.3 mg/kg enoxaparin sodium is administered to said patient by I.V. bolus before initiation of PCI if more than eight hours have passed since the patient's previous subcutaneous dose of enoxaparin sodium; wherein said treatment further comprises administering fibrinolytic therapy comprising administering one or more fibrinolytic selected from streptokinase, alteplase, tenecteplase, and reteplase; wherein said treatment further comprises administering about 150 mg to about 325 mg of non-enteric coated aspirin orally or about 500 mg intravenously after said patient is identified with ST-segment elevation myocardial infarction, and administration of doses of between about 75 to about 325 mg (coated or uncoated) once daily thereafter for a minimum of 30 days; and wherein said treatment comprises prevention of one or more of mortality, myocardial re-infarction, myocardial ischemia, stroke, or severe congestive heart failure.

[044] In the articles of manufacture of the invention described above, the enoxaparin sodium may be contained within, for example, a syringe or vial.

[045] In the articles of manufacture of the invention described above, the enoxaparin sodium and the instructions may be contained within a package. If the enoxaparin sodium is also contained within a syringe or vial, the syringe or vial may be contained within the package.

[046] The invention includes methods of treatment comprising administration of enoxaparin sodium in conjunction with fibrinolytic therapy with, for example, streptokinase, alteplase, tenecteplase, or reteplase. Administration of streptokinase, alteplase, tenecteplase, or reteplase is in accordance with their approved, prescribed or recommended administration.

[047] In embodiments of the invention in which enoxaparin sodium and a fibrinolytic therapy are both administered to a patient to treat ST-segment elevation myocardial infarction, administration of the enoxaparin sodium and the fibrinolytic therapy will be coordinated and may be administered concomitantly. One of skill in the art will recognize that appropriate coordination can be achieved in many ways, and will readily recognize how to determine which may be desirable for a particular indication. In a non-limiting example of how enoxaparin sodium and a fibrinolytic therapy may be coordinated, fibrinolytic therapy is initiated in a patient with ST-segment elevation myocardial infarction within six hours following the onset of symptoms. Enoxaparin sodium administration is then initiated from 15 minutes before the initiation of the fibrinolytic therapy to 30 minutes following the initiation of fibrinolytic therapy.

[048] The methods of the invention may prevent one or more of mortality, myocardial re-infarction, myocardial ischemia, stroke, or severe congestive heart failure for a period following initiation of therapy according to a method of the invention for one or more of 8 hours, 1 day, 2 days, 1-5 days, 2-10 days, 5-15 days, 10-20 days, 15-30 days, or greater than 30 days. In an embodiment of the invention, therapy by a method of the invention prevents one or more of mortality, myocardial re-infarction, myocardial ischemia, stroke, or severe congestive heart failure as measured at 30 days following initiation of therapy.

[049] Because patients 75 years of age or older frequently exhibit renal impairment, the methods and articles of manufacture of the invention disclosed herein may be generally applicable to patients with renal impairment, independently of patient age. Accordingly, in further embodiments of the invention a patient with renal impairment is substituted for a patient 75 years of age or older in the disclosed methods and articles of manufacture described herein.

[050] It will be readily apparent to one of ordinary skill in the relevant arts that other suitable modifications and adaptations to the methods and applications described herein are suitable and may be made without departing from the scope of the invention or any embodiment thereof. Having now described the present invention in detail, the same will be more clearly understood by reference to the following examples of the invention, included herewith for purposes of illustration only and are not intended to be limiting of the invention.

### EXAMPLES

### Example 1: Administration of Enoxaparin Sodium to Treat ST-Segment Elevation Myocardial Infarction in a Human Patient 75 years of Age or Older

[051] A patient with ST-Segment Elevation Myocardial Infarction who is 75 years of age or older is treated by a method comprising administering a dose of .75 mg per kg of the patient's body weight of enoxaparin sodium by subcutaneous injection approximately every twelve hours, for approximately 8 days or until discharge from the hospital, whichever is less. The first two doses of the enoxaparin sodium are 75 mg per kg of body weight, or 75 mg, whichever is less. The treatment also includes administration of fibrinolytic therapy comprising administering streptokinase, alteplase, tenecteplase, or reteplase. As a result of the therapy, the patient experiences a reduced risk of one or more of mortality, myocardial re-infarction, myocardial ischemia, stroke, or severe congestive heart failure.

### Example 2: Administration of Enoxaparin Sodium to Treat ST-Segment Elevation Myocardial Infarction in a Human Patient 75 years of Age or Older Wherein the Patient is Determined to have Severe Renal Impairment

[052] A patient with ST-Segment Elevation Myocardial Infarction who is 75 years of age or older is treated by a method comprising administering a dose of .75 mg per kg of the patient's body weight of enoxaparin sodium by subcutaneous injection approximately every twelve hours. The first two doses of the enoxaparin sodium are .75 mg per kg of body weight, or 75 mg, whichever is less. The treatment also includes administration of fibrinolytic therapy comprising administering streptokinase, alteplase, tenecteplase, or reteplase. The treatment further comprises determining the renal function of the patient and, if the patient is determined to have severe renal impairment, adjusting the dose of enoxaparin administered to said patient to 1 mg per kg of body weight of enoxaparin sodium administered to the patient approximately every 24 hours throughout the remainder of the therapeutic dosing period. As a result of the therapy, the patient experiences a reduced risk of one or more of mortality, myocardial re-infarction, myocardial ischemia, stroke, or severe congestive heart failure.

### Example 3: Administration of Enoxaparin Sodium to Treat ST-Segment Elevation Myocardial Infarction in a Human Patient 75 years of Age or Older to undergo PCI

[053] A patient with ST-Segment Elevation Myocardial Infarction who is 75 years of age or older is treated by a method comprising administering a dose of .75 mg per kg of the patient's body weight of enoxaparin sodium by subcutaneous injection approximately every twelve hours. The first two doses of the enoxaparin sodium are .75 mg per kg of body weight, or 75 mg, whichever is less. The treatment also includes administration of fibrinolytic therapy comprising administering streptokinase, alteplase, tenecteplase, or reteplase. The therapeutic dosing period is until approximately twelve hours or less before the patient undergoes PCI. In such a patient, a dose of 0.3 mg/kg enoxaparin sodium may be administered by I.V. bolus before initiation of PCI if more than eight hours have passed since the patient's previous subcutaneous dose of enoxaparin sodium. As a result of the therapy, the patient experiences a reduced risk of one or more of mortality, myocardial re-infarction, myocardial ischemia, stroke, or severe congestive heart failure.

[054] While the administration of enoxaparin sodium to treat ST-segment elevation myocardial infarction in a human patient 75 years of age or older has been described in connection with certain embodiments, it is not intended to limit the invention to the particular forms set forth, but on the contrary, it is intended to cover such alternatives, modifications and equivalents as may be included within the spirit and scope of the invention as defined by the following claims.

## Claims

1. A method for treating ST-segment elevation myocardial infarction in a human patient 75 years of age or older, comprising administering to said patient a dose of about .75 mg per kg of body weight of enoxaparin sodium by subcutaneous injection approximately every twelve hours for a therapeutic dosing period and without administering a GP IIb/IIIa inhibitor during said therapeutic dosing period.

2. A method as recited in claim 1, further comprising administering fibrinolytic therapy to said patient.

3. A method as recited in claim 2, wherein said fibrinolytic therapy comprises administering one or more fibrinolytic selected from streptokinase, alteplase, tenecteplase, and reteplase.

4. A method as recited in claim 3, wherein said fibrinolytic therapy comprises administering streptokinase.

5. A method as recited in claim 3, wherein said fibrinolytic therapy comprises administering alteplase.

6. A method as recited in claim 3, wherein said fibrinolytic therapy comprises administering tenecteplase.

7. A method as recited in claim 3, wherein said fibrinolytic therapy comprises administering reteplase.

8. A method as recited in claim 2, wherein the first dose of enoxaparin sodium is administered from approximately 15 minutes before to approximately 30 minutes after the initiation of fibrinolytic therapy.

9. A method as recited in claim 1, wherein each of the first two doses of enoxaparin sodium are .75 mg per kg of body weight, or 75 mg, whichever is less.

10. A method as recited in claim 1, further comprising administration of about 150 mg to about 325 mg of non-enteric coated aspirin orally or about 500 mg intravenously after said patient is identified with ST-segment elevation myocardial infarction, and administration of doses of between about 75 to about 325 mg (coated or uncoated) once daily thereafter for a minimum of 30 days.

11. A method as recited in claim 1, wherein said therapeutic dosing period is approximately 8 days or until discharge from the hospital, whichever is less.

12. A method as recited in claim 1, wherein said therapeutic dosing period is for a minimum of three doses.

13. The method as recited in claim 1, wherein said therapeutic dosing period is until approximately twelve hours our less prior to PCI therapy.

14. A method as recited in claim 13, further comprising administering a dose of 0.3 mg/kg enoxaparin sodium to said patient by I.V. bolus before initiation of PCI if more than eight hours have passed since the patient's previous subcutaneous dose of enoxaparin sodium.

15. A method as recited in claim 1, wherein said treatment comprises prevention of one or more of mortality, myocardial re-infarction, myocardial ischemia, stroke, or severe congestive heart failure.

16. A method as recited in claim 15, wherein said treatment comprises prevention of mortality.

17. A method as recited in claim 15, wherein said treatment comprises prevention of myocardial re-infarction.

18. A method as recited in claim 15, wherein said treatment comprises prevention of myocardial ischemia.

19. A method as recited in claim 15, wherein said treatment comprises prevention of stroke.

20. A method as recited in claim 15, wherein said treatment comprises prevention of severe congestive heart failure.

21. A method for treating ST-segment elevation myocardial infarction in a human patient 75 years of age or older, comprising administering to said patient at least one dose of .75 mg per kg of body weight of enoxaparin sodium by subcutaneous injection, determining the renal function of the patient and, if the patient is determined to have severe renal impairment, adjusting the dose of enoxaparin administered to said patient to 1 mg per kg of body weight of enoxaparin sodium administered to the patient approximately every 24 hours throughout the remainder of a therapeutic dosing period.

22. A method as recited in claim 21, further comprising administering fibrinolytic therapy to said patient.

23. A method as recited in claim 22, wherein said fibrinolytic therapy comprises administering one or more fibrinolytic selected from streptokinase, alteplase, tenecteplase, and reteplase.

24. A method as recited in claim 21, wherein a first dose of enoxaparin sodium is administered from 15 minutes before to 30 minutes after the initiation of fibrinolytic therapy.

25. A method as recited in claim 21, further comprising administering a dosage of 0.3 mg/kg enoxaparin sodium to said patient by I.V. bolus before initiation of PCI if more than eight hours have passed since the patient's previous subcutaneous dose of enoxaparin sodium.

26. A method for treating ST-segment elevation myocardial infarction in a human patient 75 years of age or older, comprising administering to said patient a dose of .75 mg per kg of body weight of enoxaparin sodium by subcutaneous injection approximately every twelve hours, for 8 days or until discharge from the hospital, whichever is less; wherein each of the first two doses of enoxaparin sodium are .75 mg per kg of body weight, or 75 mg, whichever is less; wherein said treatment further comprises administering fibrinolytic therapy comprising administering one or more fibrinolytic selected from streptokinase, alteplase, tenecteplase, and reteplase; wherein said treatment further comprises administering about 150 mg to about 325 mg of non-enteric coated aspirin orally or about 500 mg intravenously after said patient is identified with ST-segment elevation myocardial infarction, and administration of aspirin doses of between about 75 to about 325 mg (coated or uncoated) once daily thereafter for a minimum of 30 days.

27. A method as recited in claim 26, wherein said treatment further comprises prevention of one or more of mortality, myocardial re-infarction, myocardial ischemia, stroke, or severe congestive heart failure.

28. A method for treating ST-segment elevation myocardial infarction in a human patient 75 years of age or older, comprising administering to said patient a dose of .75 mg per kg of body weight of enoxaparin sodium by subcutaneous injection approximately every twelve hours for a therapeutic dosing period; wherein each of the first two doses of enoxaparin sodium are .75 mg per kg of body weight, or 75 mg, whichever is less; determining the renal function of the patient and, if the patient is determined to have severe renal impairment, adjusting the dose of enoxaparin administered to said patient to 1 mg per kg of body weight of enoxaparin sodium administered to the patient approximately every 24 hours throughout the remainder of a therapeutic dosing period; wherein said treatment further comprises administering fibrinolytic therapy comprising administering one or more fibrinolytic selected from streptokinase, alteplase, tenecteplase, and reteplase; wherein said treatment further comprises administering about 150 mg to about 325 mg of non-enteric coated aspirin orally or about 500 mg intravenously as soon as said patient is identified with ST-segment elevation myocardial infarction, and administration of doses of between about 75 to about 325 mg (coated or uncoated) once daily thereafter for a minimum of 30 days.

29. A method as recited in claim 28, wherein said treatment further comprises prevention of one or more of mortality, myocardial re-infarction, myocardial ischemia, stroke, or severe congestive heart failure.

30. A method for treating ST-segment elevation myocardial infarction in a human patient 75 years of age or older, comprising administering to said patient a dose of .75 mg per kg of body weight of enoxaparin sodium by subcutaneous injection approximately every twelve hours until the patient undergoes PCI; wherein each of the first two doses of enoxaparin sodium are .75 mg per kg of body weight, or 75 mg, whichever is less; wherein a dosage of 0.3 mg/kg enoxaparin sodium is administered to said patient by I.V. bolus before initiation of PCI if more than eight hours have passed since the patient's previous subcutaneous dose of enoxaparin sodium; wherein said treatment further comprises administering fibrinolytic therapy comprising administering one or more fibrinolytic selected from streptokinase, alteplase, tenecteplase, and reteplase; wherein said treatment further comprises administering about 150 mg to about 325 mg of non-enteric coated aspirin orally or about 500 mg intravenously as soon as said patient is identified with ST-segment elevation myocardial infarction, and administration of doses of between about 75 to about 325 mg (coated or uncoated) once daily thereafter for a minimum of 30 days.

31. A method as recited in claim 30, wherein said treatment further comprises prevention of one or more of mortality, myocardial re-infarction, myocardial ischemia, stroke, or severe congestive heart failure.

32. An article of manufacture for use in connection with treating ST-segment elevation myocardial infarction in a human patient 75 years of age or older, comprising enoxaparin sodium contained within a syringe and instructions for the use of said enoxaparin sodium, said instructions being designed to achieve administration to said patient of about .75 mg per kg of body weight of enoxaparin sodium by subcutaneous injection approximately every twelve hours for a therapeutic dosing period.

33. An article of manufacture as recited in claim 32, wherein said instructions are designed to achieve administration to said patient of fibrinolytic therapy.

34. An article of manufacture as recited in claim 33, wherein said fibrinolytic therapy comprises administering one or more fibrinolytic selected from streptokinase, alteplase, tenecteplase, and reteplase.

35. An article of manufacture as recited in claim 34, wherein said fibrinolytic therapy comprises administering streptokinase.

36. An article of manufacture as recited in claim 34, wherein said fibrinolytic therapy comprises administering alteplase.

37. An article of manufacture as recited in claim 34, wherein said fibrinolytic therapy comprises administering tenecteplase.

38. An article of manufacture as recited in claim 34, wherein said fibrinolytic therapy comprises administering reteplase.

39. An article of manufacture as recited in claim 33, wherein the first dose of enoxaparin sodium is administered from approximately 15 minutes before to approximately 30 minutes after the initiation of fibrinolytic therapy.

40. An article of manufacture as recited in claim 32, wherein each of the first two doses of enoxaparin sodium are .75 mg per kg of body weight, or 75 mg, whichever is less.

41. An article of manufacture as recited in claim 32, wherein said instructions are further designed to achieve administration to said patient of about 150 mg to about 325 mg of non-enteric coated aspirin orally or about 500 mg intravenously after said patient is identified with ST-segment elevation myocardial infarction, and administration of aspirin doses of between about 75 to about 325 mg (coated or uncoated) once daily thereafter for a minimum of 30 days.

42. An article of manufacture as recited in claim 32, wherein said therapeutic dosing period is approximately 8 days or until discharge from the hospital, whichever is less.

43. An article of manufacture as recited in claim 32, wherein said therapeutic dosing period is for a minimum of three doses.

44. An article of manufacture as recited in claim 32, wherein said therapeutic dosing period is until twelve hours our less prior to PCI therapy.

45. An article of manufacture as recited in claim 44, wherein said instructions are designed to achieve administration to said patient of a dosage of 0.3 mg/kg enoxaparin sodium to said patient by I.V. bolus before initiation of PCI if more than eight hours have passed since the patient's previous subcutaneous dose of enoxaparin sodium.

46. An article of manufacture as recited in claim 32, wherein said treatment comprises prevention of one or more of mortality, myocardial re-infarction, myocardial ischemia, stroke, or severe congestive heart failure.

47. An article of manufacture as recited in claim 46, wherein said treatment comprises prevention of mortality.

48. An article of manufacture as recited in claim 46, wherein said treatment comprises prevention of myocardial re-infarction.

49. An article of manufacture as recited in claim 46, wherein said treatment comprises prevention of myocardial ischemia.

50. An article of manufacture as recited in claim 46, wherein said treatment comprises prevention of stroke.

51. An article of manufacture as recited in claim 46, wherein said treatment comprises prevention of severe congestive heart failure.

52. An article of manufacture as recited in claim 32, wherein said instructions are designed to achieve administration of enoxaparin sodium to said patient without a GP IIb/IIIa inhibitor being administered to said patient during the therapeutic dosing period.

53. An article of manufacture as recited in claim 32, wherein said instructions are further designed to achieve determining the renal function of the patient and, if the patient is determined to have severe renal impairment, adjusting the dose of enoxaparin administered to said patient to 1 mg per kg of body weight of enoxaparin sodium administered to the patient approximately every 24 hours throughout the remainder of the therapeutic dosing period.

54. An article of manufacture for use in connection with treating ST-segment elevation myocardial infarction in a human patient 75 years of age or older, comprising enoxaparin sodium contained within a syringe and instructions for the use of said enoxaparin sodium, said instructions being designed to achieve administration to said patient of .75 mg per kg of body weight of enoxaparin sodium by subcutaneous injection approximately every twelve hours, for 8 days or until discharge from the hospital, whichever is less; wherein each of the first two doses of enoxaparin sodium are .75 mg per kg of body weight, or 75 mg, whichever is less; wherein said treatment further comprises administering fibrinolytic therapy comprising administering one or more fibrinolytic selected from streptokinase, alteplase, tenecteplase, and reteplase; wherein said treatment further comprises administering about 150 mg to about 325 mg of non-enteric coated aspirin orally or about 500 mg intravenously after said patient is identified with ST-segment elevation myocardial infarction, and administration of doses of between about 75 to about 325 mg (coated or uncoated) once daily thereafter for a minimum of 30 days.

55. An article of manufacture as recited in claim 54, wherein said treatment further comprises prevention of one or more of mortality, myocardial re-infarction, myocardial ischemia, stroke, or severe congestive heart failure.

56. An article of manufacture for use in connection with treating ST-segment elevation myocardial infarction in a human patient 75 years of age or older, comprising enoxaparin sodium contained within a syringe and instructions for the use of said enoxaparin sodium, said instructions being designed to achieve administration to said patient of .75 mg per kg of body weight of enoxaparin sodium subcutaneous injection approximately every twelve hours for a therapeutic dosing period; wherein each of the first two doses of enoxaparin sodium are .75 mg per kg of body weight, or 75 mg, whichever is less; wherein said instructions are further designed to achieve determining the renal function of the patient and, if the patient is determined to have severe renal impairment, adjusting the dose of enoxaparin administered to said patient to 1 mg per kg of body weight of enoxaparin sodium administered to the patient approximately every 24 hours throughout the remainder of the therapeutic dosing period; wherein said treatment further comprises administering fibrinolytic therapy comprising administering one or more fibrinolytic selected from streptokinase, alteplase, tenecteplase, and reteplase; wherein said treatment further comprises administering about 150 mg to about 325 mg of non-enteric coated aspirin orally or about 500 mg intravenously as soon as said patient is identified with ST-segment elevation myocardial infarction, and administration of doses of between about 75 to about 325 mg (coated or uncoated) once daily thereafter for a minimum of 30 days.

57. An article of manufacture as recited in claim 56, wherein said treatment further comprises prevention of one or more of mortality, myocardial re-infarction, myocardial ischemia, stroke, or severe congestive heart failure.

58. An article of manufacture for use in connection with treating ST-segment elevation myocardial infarction in a human patient 75 years of age or older, comprising enoxaparin sodium contained within a syringe and instructions for the use of said enoxaparin sodium, said instructions being designed to achieve administration to said patient of .75 mg per kg of body weight of enoxaparin sodium by subcutaneous injection approximately every twelve hours until the patient undergoes PCI; wherein each of the first two doses of enoxaparin sodium are .75 mg per kg of body weight, or 75 mg, whichever is less; wherein a dosage of 0.3 mg/kg enoxaparin sodium is administered to said patient by I.V. bolus before initiation of PCI if more than eight hours have passed since the patient's previous subcutaneous dose of enoxaparin sodium; wherein said treatment further comprises administering fibrinolytic therapy comprising administering one or more fibrinolytic selected from streptokinase, alteplase, tenecteplase, and reteplase; wherein said treatment further comprises administering about 150 mg to about 325 mg of non-enteric coated aspirin orally or about 500 mg intravenously as soon as said patient is identified with ST-segment elevation myocardial infarction, and administration of doses of between about 75 to about 325 mg (coated or uncoated) once daily thereafter for a minimum of 30 days.

59. An article of manufacture as recited in claim 58, wherein said treatment further comprises prevention of one or more of mortality, myocardial re-infarction, myocardial ischemia, stroke, or severe congestive heart failure.

60. A method for treating ST-segment elevation myocardial infarction in a human patient 75 years of age or older, comprising administering to said patient a dose of about .75 mg per kg of body weight of enoxaparin sodium by subcutaneous injection approximately every twelve hours for a therapeutic dosing period, wherein said therapeutic dosing period comprises a minimum of three doses.

61. A method as recited in claim 60, further comprising administering fibrinolytic therapy to said patient.

62. A method as recited in claim 61, wherein said fibrinolytic therapy comprises administering one or more fibrinolytic selected from streptokinase, alteplase, tenecteplase, and reteplase.

63. A method as recited in claim 62, wherein said fibrinolytic therapy comprises administering streptokinase.

64. A method as recited in claim 62, wherein said fibrinolytic therapy comprises administering alteplase.

65. A method as recited in claim 62, wherein said fibrinolytic therapy comprises administering tenecteplase.

66. A method as recited in claim 62, wherein said fibrinolytic therapy comprises administering reteplase.

67. A method as recited in claim 61, wherein the first dose of enoxaparin sodium is administered from 15 minutes before to 30 minutes after the initiation of fibrinolytic therapy.

68. A method as recited in claim 60, wherein each of the first two doses of enoxaparin sodium are .75 mg per kg of body weight, or 75 mg, whichever is less.

69. A method as recited in claim 60, further comprising administration of about 150 mg to about 325 mg of non-enteric coated aspirin orally or about 500 mg intravenously as soon as said patient is identified with ST-segment elevation myocardial infarction, and administration of doses of between about 75 to about 325 mg (coated or uncoated) once daily thereafter for a minimum of 30 days.

70. A method as recited in claim 60, wherein said therapeutic dosing period is approximately 8 days or until discharge from the hospital, whichever is less.

71. The method as recited in claim 60, wherein said therapeutic dosing period is until twelve hours our less prior to PCI therapy.

72. A method as recited in claim 71, further comprising administering a dosage of 0.3 mg/kg enoxaparin sodium to said patient by I.V. bolus before initiation of PCI if more than eight hours have passed since the patient's previous subcutaneous dose of enoxaparin sodium and the initiation of PCI.

73. A method as recited in claim 60, wherein said treatment comprises prevention of one or more of mortality, myocardial re-infarction, myocardial ischemia, stroke, or severe congestive heart failure.

74. A method as recited in claim 73, wherein said treatment comprises prevention of mortality.

75. A method as recited in claim 73, wherein said treatment comprises prevention of myocardial re-infarction.

76. A method as recited in claim 73, wherein said treatment comprises prevention of myocardial ischemia.

77. A method as recited in claim 73, wherein said treatment comprises prevention of stroke.

78. A method as recited in claim 73, wherein said treatment comprises prevention of severe congestive heart failure.

79. A method for treating ST-segment elevation myocardial infarction in a human patient with renal impairment, comprising administering to said patient a dose of about .75 mg per kg of body weight of enoxaparin sodium by subcutaneous injection approximately every twelve hours for a therapeutic dosing period.

80. A method as recited in claim 79, wherein said treatment comprises prevention of one or more of mortality, myocardial re-infarction, myocardial ischemia, stroke, or severe congestive heart failure.

81. A method as recited in claim 79, further comprising administering fibrinolytic therapy to said patient.

82. A method as recited in claim 81, wherein said fibrinolytic therapy comprises administering one or more fibrinolytic selected from streptokinase, alteplase, tenecteplase, and reteplase.

83. A method as recited in claim 79, wherein the first dose of enoxaparin sodium is administered from approximately 15 minutes before to approximately 30 minutes after the initiation of fibrinolytic therapy.

84. A method as recited in claim 79, wherein each of the first two doses of enoxaparin sodium are .75 mg per kg of body weight, or 75 mg, whichever is less.

85. A method as recited in claim 79, further comprising administration of about 150 mg to about 325 mg of non-enteric coated aspirin orally or about 500 mg intravenously after said patient is identified with ST-segment elevation myocardial infarction, and administration of doses of between about 75 to about 325 mg (coated or uncoated) once daily thereafter for a minimum of 30 days.

86. A method as recited in claim 79, wherein said therapeutic dosing period is approximately 8 days or until discharge from the hospital, whichever is less.

87. A method as recited in claim 79, wherein said therapeutic dosing period is for a minimum of three doses.

88. A method as recited in claim 79, wherein said therapeutic dosing period is until approximately twelve hours our less prior to PCI therapy.

89. A method as recited in claim 88, further comprising administering a dose of 0.3 mg/kg enoxaparin sodium to said patient by I.V. bolus before initiation of PCI if more than eight hours have passed since the patient's previous subcutaneous dose of enoxaparin sodium.
